(19) Europäisches Patentamt
European Patent Office
Office européen des brevets

(11) Numéro de publication : **0 676 203 A1**

(12) # DEMANDE DE BREVET EUROPEEN

(21) Numéro de dépôt : **95400764.7**

(22) Date de dépôt : **06.04.95**

(51) Int. Cl.$^6$ : **A61K 31/565,** A61K 31/575, A61K 31/56

(30) Priorité : **08.04.94 FR 9404156**

(43) Date de publication de la demande : **11.10.95 Bulletin 95/41**

(84) Etats contractants désignés : **AT BE CH DE DK ES FR GB GR IE IT LI LU NL PT SE**

(71) Demandeur : **ROUSSEL UCLAF
102 Route de Noisy
F-93230 Romainville (FR)**

(72) Inventeur : **Petit, Francis
111, rue Jules Ferry
F-92700 Colombes (FR)**
Inventeur : **Philibert, Daniel
16, rue Chevalier
F-94210 La Varenne Saint Hilaire (FR)**
Inventeur : **Ulmann, André
63, rue de la Colonie
F-75013 Paris (FR)**

(74) Mandataire : **Vieillefosse, Jean-Claude et al
ROUSSEL UCLAF
Département des Brevets
102, Route de Noisy
F-93235 Romainville (FR)**

(54) **Composition pharmaceutiques des composés ayant une activité anti-glucocorticoide pour la prévention ou traitement des manifestations liées au syndrome de sevrage spontané ou précipité des narcotiques.**

(57) L'invention a pour objet l'application des composés ayant une activité antiglucocorticoïde pour la préparation de médicaments destinés à la prévention ou au traitement des manifestations liées au syndrome de sevrage spontané ou précipité des narcotiques ou mélanges de narcotiques et les compositions les renfermant.

EP 0 676 203 A1

La présente invention concerne l'application des composés ayant une activité antiglucocorticoïde pour la préparation de médicaments destinés à la prévention ou au traitement des manifestations liées au syndrome de sevrage spontané ou précipité des narcotiques ou mélanges de narcotiques et les compositions les renfermant.

Les produits possédant une activité antiglucocorticoïde sont connus comme pouvant être utilisés comme médicaments pour lutter principalement contre les effets secondaires des glucocorticoïdes ; ils permettent de lutter également contre les troubles dus à une hypersécrétion de glucocorticoïdes notamment contre le vieillissement en général et plus particulièrement contre l'hypertension, le glaucome, l'athérosclérose, l'ostéoporose, le diabète, l'obésité ainsi que la dépression de l'immunité et l'insomnie.

La demanderesse a mis en évidence l'application nouvelle et inattendue de ces produits, énoncée plus haut.

Il a été précédemment montré que les glucocorticoïdes (type dexaméthasone) antagonisent l'activité analgésique de la morphine alors qu'un antagoniste des glucocorticoïdes, type 17β-hydroxy 11β-(4-diméthylaminophényl) 17α-(prop-1-ynyl) estra 4,9-dièn-3-one, ou une surrénalectomie potentialise cette activité (Capasso et al. Life Science 51 139 (1992), Ratka et al. Neuroendocrinology 49 439 (1988) Pieretti et al. Gen. Pharmacol. 22 929 (1991)).

Cependant, à la connaissance de la demanderesse, personne n'a mis en évidence l'activité d'un antiglucocorticoïde vis-à-vis des effets indésirables des opiacés et en particulier de l'induction d'un état de dépendance physique ou psychique et du syndrome de sevrage qui est associé à cet état. Ces phénomènes de dépendance et de sevrage impliquent des mécanismes centraux complexes, multiples et différents de ceux qui sont observés dans l'activité analgésique des opiacés.

D'autre part, des données récentes ont été rapportées sur le rôle important que pouvaient jouer les glucocorticoïdes endogènes dans les manifestations du sevrage narcotique, de même que dans les phénomènes de dépendance induits par les opiacés ou la cocaïne. Ainsi, un hypercortisolisme a été observé chez l'homme au cours d'essais cliniques, lors d'un sevrage spontané ou précipité par la naloxone consécutif à la prise d'héroïne ou de morphine (Cami et al. Br. J. Addict 87 1145 (1992), Higgins et al. Drug Alcohol Depend. 30 13 (1992). D'autres éléments rapportés chez l'animal montrent, une activation de l'axe hypothalamo-surrénalien par la cocaïne (Borowsky and Kuhn, J. Pharmacol. Exp. Ther. (1991) 256, 204) administrée en traitement aigü ou répété avec une augmentation des taux plasmatiques de corticostérone et d'ACTH (Moldow and Fischman, Peptides 8 819 (1987), Yang et al. Pharmacol. Biochem Behau. 41 643 (1992), Saphier et al. Neuroendocrinology 57 54 (1993)) consécutive à une médiation d'origine monoaminergique (dopamine par exemple). Par exemple l'implication du système dopaminergique semble confirmée par le fait que l'halopéridol et le métoclopramide (antagonistes dopaminergiques) s'opposent respectivement à l'élévation des taux de corticostérone induite par la cocaïne et au phénomène de sevrage morphinique (Ramaswamy and Bapna, Life Science 40 807 (1987)).

Ces données semblent montrer que les glucocorticoïdes endogènes pourraient intervenir dans les phénomènes de sevrage et de dépendance, au même titre que des mécanismes dopaminergiques mais à une étape plus en avant que ces derniers.

Ces différents éléments ont justifié l'étude d'antagonistes des glucocorticoïdes en particulier vis-à-vis du syndrome de sevrage morphinique précipité par la naloxone chez l'animal puisqu'aucune donnée n'est actuellement disponible sur l'activité de cette classe thérapeutique dans cet axe.

En effet, si une élévation des taux de glucocorticoïdes endogènes a été rapportée dans les phénomènes de sevrage opiacé, il n'a pas été démontré que cette augmentation pouvait avoir un retentissement physiopathologique et qu'en particulier le blocage de ces glucocorticoïdes endogènes au niveau de leurs récepteurs par un antiglucocorticoïde pouvait se traduire par un effet bénéfique sur les manifestations du syndrome de sevrage.

C'est ainsi que la demanderesse a mis en évidence une application nouvelle et inattendue des antiglucocorticoïdes.

La présente invention a donc pour objet l'application des composés ayant une activité antiglucocorticoïde pour la préparation de médicaments destinés à la prévention ou au traitement des manifestations liées au syndrome de sevrage spontané ou précipité des narcotiques ou mélanges de narcotiques.

On entend par narcotiques, toutes drogues entraînant un phénomène de dépendance physique et psychique et dont l'arrêt spontané ou précipité entraîne un syndrome de sevrage. On peut citer :

**1)** les morphinomimétiques naturels tels que :

a) les alcaloïdes de l'opium, par exemple la morphine,

b) les alcaloïdes dérivés de la morphine, par exemple l'héroïne ou la codéïne,

**2)** les morphinomimétiques de synthèse tels que :

a) les dérivés de la pipéridine, par exemple la péthidine ou

b) la méthadone et ses dérivés, par exempe la dextromoramide,

**3)** la cocaïne, ainsi que toutes les associations renfermant deux ou plusieurs de ces produits narcotiques.

La présente invention a plus spécialement pour objet l'application des composés ayant une activité antiglucocorticoïde pour la préparation de médicaments destinés à la prévention ou au traitement des manifestations liées au syndrome de sevrage spontané ou précipité des narcotiques morphinomimétiques choisis parmi l'héroïne, la morphine et la méthadone.

La présente invention a plus spécialement pour objet l'application des composés ayant une activité antiglucocorticoïde pour la préparation de médicaments destinés à la prévention ou au traitement des manifestations liées au syndrome de sevrage spontané ou précipité de la cocaïne.

La présente invention a plus précisément pour objet l'application telle que définie précédemment, caractérisée en ce que les composés ayant une activité antiglucocorticoïde répondent à la formule générale (I) :

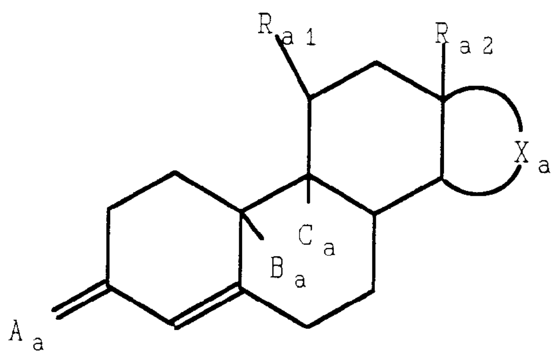

(I)

dans laquelle $R_{a1}$ représente un radical organique renfermant de 1 à 18 atomes de carbone, contenant au moins un atome d'azote, de phosphore ou de silicium, l'atome immédiatement adjacent au carbone en 11 étant un atome de carbone, $R_{a2}$ représente un radical hydrocarboné renfermant de 1 à 8 atomes de carbone, $X_a$ représente le reste d'un cycle pentagonal ou hexagonal éventuellement substitué et éventuellement porteur d'insaturation, le groupement C = $A_a$ en position 3 représente un groupement oxo, libre ou bloqué sous forme de cétal, un groupement :

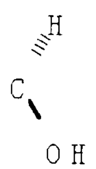 , 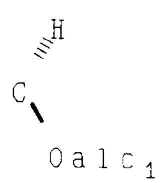 , 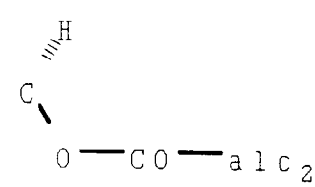 ,

un groupement C = NOH, un groupement C=NO-$alc_3$, ou un groupement $CH_2$, $alc_1$, $alc_2$ et $alc_3$ représentant un radical alkyle renfermant de 1 à 8 atomes de carbone ou un groupement aralkyle renfermant de 7 à 15 atomes de carbone et $B_a$ et $C_a$ forment ensemble une double liaison ou un pont époxyde, ainsi que leurs sels d'addition avec les acides pharmaceutiquement acceptables.

Les produits de formule générale (I) sont décrits et préparés dans le brevet européen EP 57115 et dans le certificat d'addition FR 2625505.

Les définitions des divers substituants de ladite formule ainsi que les valeurs particulières desdits substituants et l'ensemble des produits synthétisés qui sont considérés comme des produits préférés dans le cadre de la présente invention, sont définis dans le brevet européen et le certificat d'addition précités.

La présente invention a tout particulièrement pour objet l'application telle que définie précédemment des produits répondant à la formule générale (I), choisis dans la liste suivante :

- 17β-hydroxy 11β-(4-diméthylaminophényl) 17α-(prop-1-ynyl) estra 4,9-dièn-3-one (Mifépristone) ;
- N-oxyde du 21-chloro 17β-hydroxy 11β-(4-diméthyl aminophényl) 17α-19-nor-pregna-4,9-dièn-20-yn-3-one ;
- (Z) 11β-[4-(diméthylamino) phényl] 17β-hydroxy 17α-[(Z)-1-propényl] estra-4,9-dièn-3-one,

ainsi que leurs sels d'addition avec les acides pharmaceutiquement acceptables.

La présente invention a tout spécialement pour objet l'application telle que définie précédemment du produit répondant à la formule générale (I) suivant, la :

- 17β-hydroxy 11β-(4-diméthylaminophényl) 17α-(prop-1-ynyl) estra 4,9-dièn-3-one (Mifépristone).

La présente invention a plus précisément pour objet l'application telle que définie précédemment caractérisée en ce que les composés ayant une activité antiglucocorticoïde répondent à la formule générale (II) :

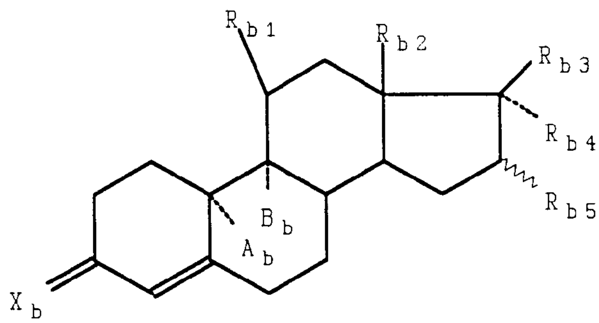

(II)

dans laquelle soit $R_{b1}$ représente un radical thiényle éventuellement substitué, un radical furyle, un radical cycloalkyle ayant de 3 à 6 atomes de carbone, un radical phényle éventuellement substitué par un ou plusieurs radicaux choisis parmi les radicaux hydroxy, halogène, trifluorométhyle, alkyle, alkoxy, alkylthio éventuellement oxydé sous forme de sulfoxyde ou de sulfone, alkényloxy ayant au plus 6 atomes de carbone et phényloxy, soit $R_{b1}$ représente un radical naphtyle ou phényl-phényle ou un radical alkyle ou alkényle portant éventuellement plusieurs insaturations et ayant au plus 6 atomes de carbone,

$R_{b2}$ représente un radical méthyle ou éthyle,

$R_{b3}$ représente un atome d'hydrogène, un radical alkyle, alkényle ou alkynyle éventuellement substitué, un radical hydroxy, acétyle, hydroxyacétyle, carboxyalkoxy ayant de 2 à 4 atomes de carbone éventuellement estérifié ou salifié, hydroxy-alkyle éventuellement estérifié,

$R_{b4}$ représente un atome d'hydrogène, un radical hydroxy ou un radical alkyle, alkényle ou alkynyle ayant au plus 12 atomes de carbone éventuellement substitué par un radical amino, alkylamino, dialkylamino, ou par un halogène, un radical alkylthio, alkoxy, trialkylsilyl ou cyano,

$R_{b5}$ représente un atome d'hydrogène ou un radical méthyle en position $\alpha$ ou $\beta$,

$X_b$ représente un atome d'oxygène ou un radical hydroxyimino ou alkoxyimino ayant de 1 à 4 atomes de carbone, en position syn ou anti ;

$A_b$ et $B_b$ représentent une fonction $\alpha$-époxy ou une seconde liaison entre les carbones 9 et 10 ;

ainsi que leurs sels d'addition avec les acides pharmaceutiquement acceptables lorsque $R_{b4}$ représente un radical comportant une fonction amino.

Les produits de formule générale (II) sont décrits et préparés dans les demandes de brevets ou brevets WO 83/03099, EP 0369881 et FR 2377418.

Les définitions des divers substituants de ladite formule ainsi que les valeurs particulières desdits substituants et l'ensemble des produits synthétisés qui sont considérés comme des produits préférés dans le cadre de la présente invention sont définis dans les demandes de brevets ou brevets précités.

La présente invention a tout particulièrement pour objet l'application telle que définie précédemment des produits répondant à la formule générale (II), choisis dans la liste suivante :

- 11β-[(4-méthylthio) phényl] 17β-hydroxy 17α-(prop-1-ynyl) estra-4,9-dièn-3-one,
- 9α,10α-époxy 17β-hydroxy 11β-[(4-méthylsulfonyl) phényl] 17α-(prop-1-ynyl) estr-4-èn-3-one.

La présente invention a plus précisément pour objet l'application telle que définie précédemment, caractérisée en ce que les composés ayant une activité antiglucocorticoïde répondent à la formule générale (III) :

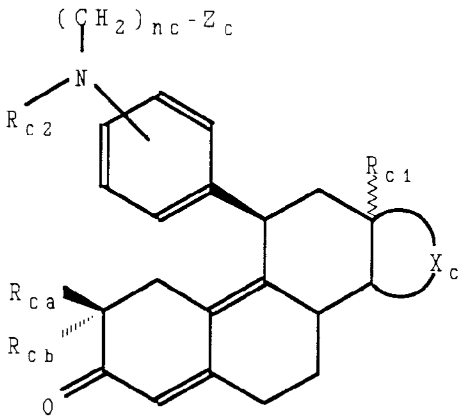

(III)

dans laquelle $R_{c1}$ représente un radical hydrocarboné aliphatique renfermant de 1 à 8 atomes de carbone, $R_{ca}$ et $R_{cb}$ identiques ou différents représentent un atome d'hydrogène, un radical alkyle renfermant de 1 à 4 atomes de carbone, $R_{c2}$ représente un atome d'hydrogène ou un radical alkyle renfermant de 1 à 12 atomes de carbone et éventuellement substitué, nc représente un entier de 1 à 6, $Z_c$ représente un groupe carboxy libre ou salifié sous forme d'un sel de métal alcalin ou alcalino-terreux, d'un sel d'ammonium ou d'un sel d'amine, $X_c$ représente le reste d'un cycle pentagonal ou hexagonal éventuellement substitué et éventuellement porteur d'une insaturation, et le trait ondulé en position 13 signifie que $R_{c1}$ peut se trouver en position alpha ou béta.

Les produits de formule générale (III) sont décrits et préparés dans le brevet européen EP 414606.

Les définitions des divers substituants de ladite formule ainsi que les valeurs particulières desdits substituants et l'ensemble des produits synthétisés qui sont considérés comme des produits préférés dans le cadre de la présente invention sont définis dans le brevet européen précité.

La présente invention a tout particulièrement pour objet l'application telle que définie précédemment des produits répondant à la formule générale (III) choisis dans la liste suivante :

- sel de sodium de l'acide [[4-[21-chloro 17β-hydroxy 3-oxo 19-nor 17α-pregna-4,9-dièn-20-yn-[11β-yl] phényl] méthylamino] acétique,
- sel de sodium de l'acide [[4-[17β-hydroxy 3-oxo 17α-(2-propényl) estra-4,9-dièn-11β-yl] phényl] méthylamino] acétique,
- [[4-[17β-hydroxy 3-oxo 17α-(1-propynyl) estra-4,9-dièn-11β-yl] phényl] méthylamino] acétate d'éthyle,
- sel de sodium de l'acide [[4-[17β-hydroxy 3-oxo 17α-(1-propynyl) estra-4,9-dièn-11β-yl] phényl] méthylamino] acétique.

La présente invention a plus précisément pour objet l'application telle que définie précédemment caractérisée en ce que les composés ayant une activité antiglucocorticoïde répondent à la formule générale (IV) :

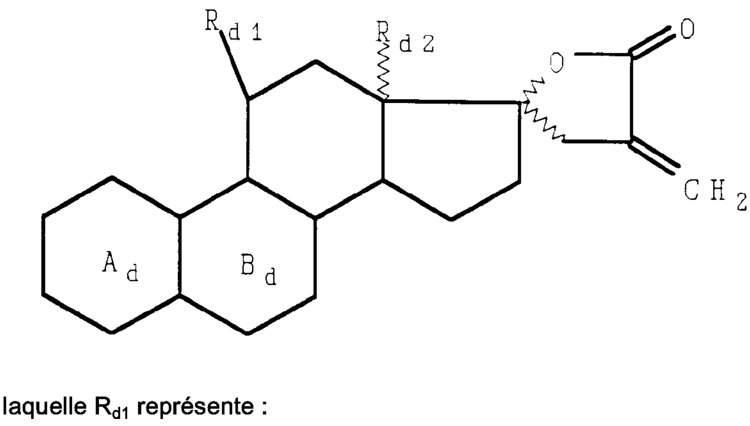

(IV)

dans laquelle $R_{d1}$ représente :

soit un radical phényle, biphényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle, chacun de ces radicaux étant éventuellement substitué par un ou plusieurs radicaux choisis parmi :

- les radicaux alkyle, alkényle ou alkynyle ayant au plus 8 atomes de carbone et éventuellement substitués par un ou plusieurs radicaux choisis parmi les radicaux hydroxyle, halogène, oxo, trialkylsilyle, alkoxy ou alkylthio, les radicaux alkyle, alkoxy et alkylthio ayant de 1 à 4 atomes de carbone,

- les radicaux alkoxy, alkényloxy ou alkylthio éventuellement oxydés sous forme de sulfoxyde ou de sulfone, tous ces radicaux ayant au plus 4 atomes de carbone,
- les atomes d'halogène,
- les radicaux trialkylsilyle, chaque radical alkyle comportant de 1 à 4 atomes de carbone,
- les radicaux hydroxyle, trifluorométhyle, acyle ayant de 1 à 6 atomes de carbone, carboxy libre estérifié ou salifié,
- le radical

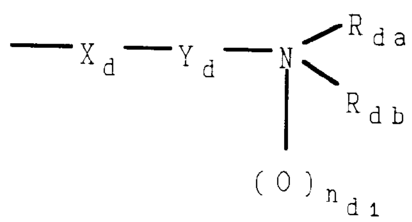

dans lequel $X_d$ représente une simple liaison, un atome d'oxygène, de soufre ou un radical

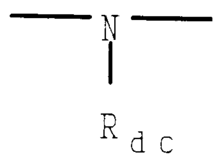

$Y_d$ représente une simple liaison, un radical alkylène, alkénylène ou alkynylène linéaire ou ramifié ayant au plus 8 atomes de carbone, $n_{d1}$ représente 0 ou 1,

$R_{da}$ et $R_{db}$ identiques ou différents représentent :

. un atome d'hydrogène,

. un radical alkyle ayant de 1 à 4 atomes de carbone éventuellement substitué par un radical carboxy libre estérifié ou salifié, un radical trialkylsilyle, chaque radical alkyle comportant de 1 à 4 atomes de carbone,

. un radical acyle ayant de 1 à 8 atomes de carbone,

ou $R_{da}$ et $R_{db}$ forment avec l'atome de carbone auquel ils sont liés un hétérocycle comportant éventuellement un autre hétéroatome choisi parmi l'oxygène, le soufre ou l'azote et éventuellement substitué par un radical alkyle ayant de 1 à 4 atomes de carbone, étant entendu que lorsque $X_d$ représente un atome de soufre, d'oxygène ou un radical

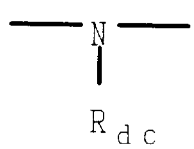

$Y_d$ ne peut pas être une simple liaison ou un radical méthylène,

$R_{dc}$ représente un atome d'hydrogène ou un radical alkyle ayant de 1 à 4 atomes de carbone,

soit $R_{d1}$ représente un radical indényle ou quinoléinyle éventuellement substitué par un radical alkyle ayant de 1 à 4 atomes de carbone et éventuellement hydrogéné,

$R_{d2}$ en position $\alpha$ ou $\beta$ représente un radical alkyle ayant de 1 à 4 atomes de carbone,

les traits ondulés en position 17 indiquent que le cycle lactonique peut se trouver dans l'une ou l'autre des positions possibles,

les cycles $A_d$ et $B_d$ représentent l'une des deux structures suivantes :

a) soit $A_d$ et $B_d$ représentent le groupement :

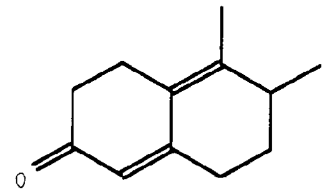

b) soit $A_d$ et $B_d$ représentent le groupement :

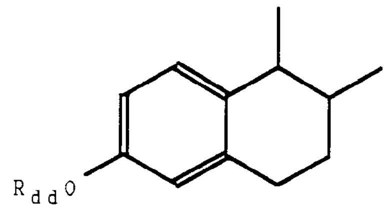

dans lequel $R_{dd}$ représente un atome d'hydrogène ou un radical alkyle ayant de 1 à 4 atomes de carbone éventuellement substitué par un ou plusieurs radicaux choisis parmi les radicaux hydroxyle et les atomes d'halogènes, ou $R_{dd}$ représente un radical arylalkyle ayant au plus 12 atomes de carbone ou un radical acyle ayant de 1 à 6 atomes de carbone, ainsi que les sels des produits de formule (IV) avec les acides et les bases pharmaceutiquement acceptables.

Les produits de formule générale (IV) sont décrits et préparés dans le brevet européen EP 0558416.

Les définitions des divers substituants de ladite formule ainsi que les valeurs particulières desdits substituants et l'ensemble des produits synthétisés qui sont considérés comme des produits préférés dans le cadre de la présente invention sont définis dans le brevet européen précité.

La présente invention a tout spécialement pour objet, l'application telle que définie précédemment, du produit répondant à la formule générale (IV) suivant :

- γ-lactone de l'acide 17β-hydroxy 11β-[4-(diméthylamino) phényl] 21-méthylène 3-oxo 19-nor 17α-pregna-4,9-diène-21-carboxylique.

La présente invention a plus précisément pour objet l'application telle que définie précédemment, caractérisée en ce que les composés ayant une activité antiglucocorticoïde répondent à la formule générale (V) :

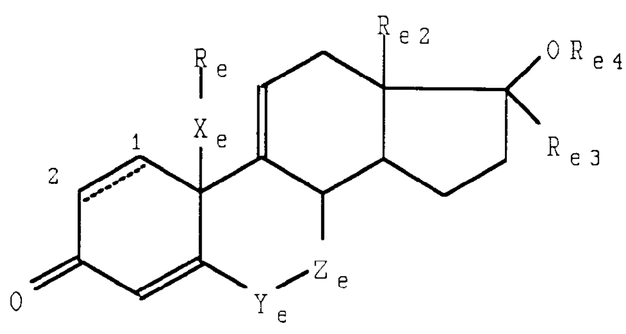

(V)

dans laquelle $X_e$ et $R_e$ sont tels que :

- soit $X_e$ représente un radical méthylène et $R_e$ représente
  ou bien un radical aryle carbocyclique ou hétérocyclique éventuellement substitué,
  ou bien un radical vinyle ou éthynyle éventuellement substitué,
- soit $X_e$ représente un atome de soufre ou une simple liaison et $R_e$ représente un radical aryle carbocyclique ou hétérocyclique éventuellement substitué,

$R_{e2}$ représente un radical méthyle ou éthyle,

$R_{e3}$ représente un radical alkyle, alkényle ou alkynyle ayant au plus 8 atomes de carbone, éventuellement substitué,

$R_{e4}$ représente un atome d'hydrogène ou un radical acyle,

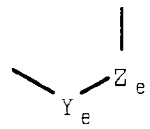

représente :
ou bien un groupement

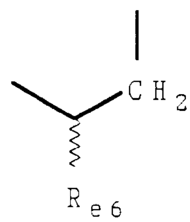

dans lequel $R_{e6}$ représente un atome d'hydrogène ou un radical méthyle en position α et/ou β,
ou bien un groupement

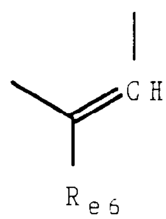

dans lequel $R_{e6}$ représente un atome d'hydrogène ou un radical méthyle,
ou bien un groupement

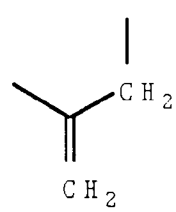

le trait pointillé en position 1(2) indique la présence éventuelle d'une seconde liaison carbone-carbone.

Les produits de formule générale (V) sont décrits et préparés dans le brevet européen EP 0188396.

Les définitions des divers substituants de ladite formule ainsi que les valeurs particulières desdits substituants et l'ensemble des produits synthétisés qui sont considérés comme des produits préférés dans le cadre de la présente invention sont définis dans le brevet européen précité.

La présente invention a tout spécialement pour objet l'application telle que définie précédemment du produit, répondant à la formule générale (V) suivant :

- 17β-hydroxy 10β-[(4-méthyl phényl) méthyl] 17α-prop-1-ynyl) estra-4,9(11)dièn-3-one,
- 10β-benzyl 17β-hydroxy 17α-(prop-1-ynyl) estra-4,9(11)dièn-3-one.

La présente invention a plus précisément pour objet l'application telle que définie précédemment, caractérisée en ce que les composés ayant une activité antiglucocorticoïde répondent à la formule générale (VI) :

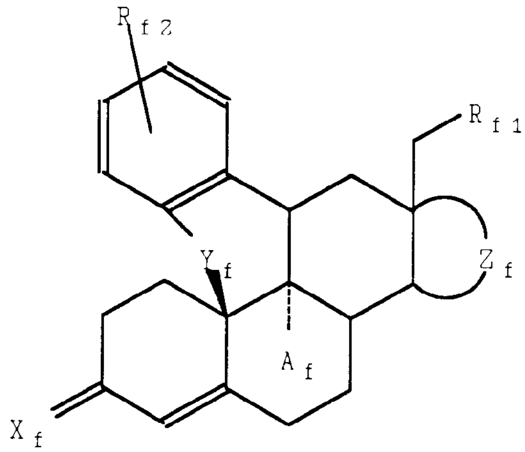

(VI)

dans laquelle :

$R_{f1}$ représente un atome d'hydrogène ou un groupement méthyle, $R_{f2}$ en position 4 ou 5 représente un atome d'hydrogène, un groupement cyano, un groupement hétéroaryle, un groupement alkyle, alkényle ou alkynyle ayant jusqu'à 20 atomes de carbone éventuellement substitué par un ou plusieurs groupement oxo, un groupement $OR_{f3}$, un groupement $SR_{f3}$ dans lesquels $R_{f3}$ représente un atome d'hydrogène ou un groupement alkyle ayant de 1 à 8 atomes de carbone, un groupement $OSO_2R_{f11}$ dans lequel $R_{f11}$ représente un groupement alkyle ayant de 1 à 4 atomes de carbone perfluoré ou un groupement $NR_{f3}R_{f4}$ dans lequel

soit $R_{f3}$ est tel que défini plus haut et $R_{f4}$ identique ou différent de $R_{f3}$ représente un atome d'hydrogène, un groupement alkyle ayant de 1 à 10 atomes de carbone, un groupement cyano ou un groupement acyle ayant de 2 à 10 atomes de carbone,

soit $R_{f3}$ et $R_{f4}$ forment ensemble avec l'azote auquel ils sont liés un hétérocycle à 5 ou 6 chaînons comportant éventuellement un second hétéroatome choisi parmi N, O et S,

$X_f$ représente un groupement céto ou oxime,

$Z_f$ représente le reste d'un cycle pentagonal ou hexagonal éventuellement substitué et éventuellement porteur d'insaturations,

$Y_f$ représente un groupement $-CH_2-$ ou un atome de soufre et $A_f$ représente un atome d'hydrogène ou représente un groupement OH dans le cas où $Y_f$ n'est pas un atome de soufre, ainsi que les sels d'addition avec les acides ou les bases pharmaceutiquement acceptables.

Les produits de formule générale (VI) sont décrits et préparés dans les brevets européens EP 283428, EP 399632 et EP 347370.

Les définitions des divers substituants de ladite formule, ainsi que les valeurs particulières desdits substituants et l'ensemble des produits synthétisés qui sont considérés comme des produits préférés dans le cadre de la présente invention sont définis dans les brevets européens précités.

La présente invention a tout -particulièrement pour objet l'application telle que définie précédemment des produits répondant à la formule générale (VI), choisis dans la liste suivante :

- 17-(3-hydroxy prop-1(Z)-ényl) 17β-hybroxy 11β,19-(4-méthylthio-0-phénylène) 4-androstèn-3-one,
- 17-(prop-1-inyl) 17β-hydroxy 11β,19-(4-diméthylamino-0-phénylène) 4-androstèn-3-one,
- 17-(3-hydroxy prop-1(Z)-ényl) 17β-hydroxy 11β,19-(4-diméthylamino-0-phénylène) 4-androstèn-3-one,
- 17-(prop-1-inyl) 17β-hydroxy 11β,19-(4-acétyl-0-phénylène) 4-androstèn-3-one,
- 9α,17β-dihydroxy 11β,19-[4-(3-pyridyl)-0-phénylène] 17-(prop-1-inyl) 4-androstèn-3-one,
- 10β,11β-(5-diméthylamino-0-phénylènethio) 17β-hydroxy 17α-(3-hydroxyprop-1(Z)-ényl) estr-4-èn-3-one.

La présente invention a plus précisément pour objet l'application telle que définie précédemment, caractérisée en ce que les composés ayant une activité antiglucocorticoïde répondent à la formule générale (VII) :

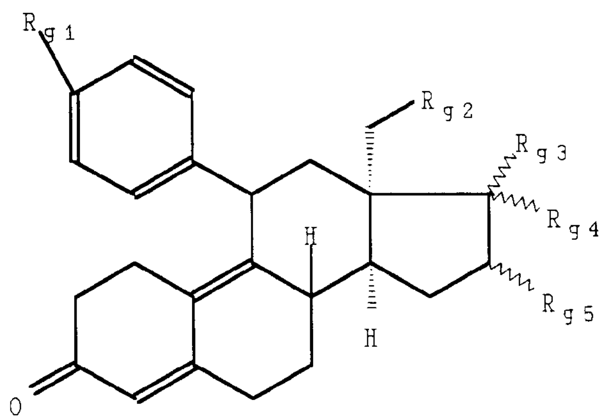

(VII)

dans laquelle :

$R_{g1}$ représente ou bien le groupement $NR_{gI}R_{gII}$, éventuellement oxydé sous forme de N-oxyde, dans lequel soit $R_{gI}$ et $R_{gII}$ identiques ou différents représentent un atome d'hydrogène ou un groupement alkyle ayant de 1 à 4 atomes de carbone, soit $R_{gI}$ et $R_{gII}$ forment ensemble avec l'atome d'azote auquel ils sont liés un hétérocycle à 5 ou 6 chaînons comportant éventuellement un second hétéroatome choisi parmi N, O et S,

ou bien le groupement $OR_{gIII}$ dans lequel $R_{gIII}$ représente un groupement méthyle, éthyle, propyle, méthoxyphényle, alkyle ou β-diméthylamino éthyle,

$R_{g2}$ représente un atome d'hydrogène, un groupement méthyle ou éthyle,

soit $R_{g3}$ représente un groupement $-(CH_2)_{ng}-CH_3$ dans lequel ng est égal à 0 à 4, un groupement $-(CH_2)_{ng}-CH_2-O-R_{gIV}$ ou $-(CH_2)_{ng}-CH_2-S-R_{gIV}$ dans lequel ng est égal à 0 à 4 et $R_{gIV}$ représente un atome d'hydrogène ou un groupement alkyle ayant de 1 à 4 atomes de carbone, un groupement $-CH=CH-(CH_2)_{ng}-OR_{gV}$, dans lequel ng est égal à 0 à 4 et $R_{gV}$ représente un atome d'hydrogène, un groupement alkyle ou alkanoyle ayant chacun de 1 à 4 atomes de carbone, un groupement $C{\equiv}C-X_g$, dans lequel $X_g$ représente un atome d'hydrogène, un groupement alkyle ayant de 1 à 4 atomes de carbone ou un halogène, un groupement $(CH_2)_{ng}-CH_2CN$ dans lequel ng est égal à 0 à 3 ou un groupement $-CO-CH_2-Y_g$ dans lequel Y représente un atome d'hydrogène ou $OR_{gV}$, $R_{gV}$ étant tel que défini précédemment,

et $R_{g4}$ représente un groupement hydroxy, alkoxy ou alkényloxy ayant chacun de 1 à 4 atomes de carbone,

soit $R_{g3}$ et $R_{g4}$ forment ensemble le cycle

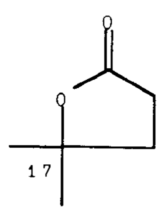

avec $R_{g3}$ en position α et $R_{g4}$ en position β ou $R_{g3}$ en position β et $R_{g4}$ en position α

et $R_{g5}$ représente un atome d'hydrogène ou un groupement alkyle ayant de 1 à 4 atomes de carbone en position α ou β, ainsi que leurs sels d'addition avec les acides et les bases pharmaceutiquement acceptables.

Les produits de formule générale (VII) sont décrits et préparés dans le brevet européen EP 0129499.

Les définitions des divers substituants de ladite formule ainsi que les valeurs particulières desdits substituants et l'ensemble des produits synthétisés qui sont considérés comme des produits préférés dans le cadre de la présente invention sont définis dans le brevet européen précité.

La présente invention a tout spécialement pour objet l'application telle que définie précédemment, du produit répondant à la formule générale (VII), suivant :

- 11β-[4-(diméthylamino) phényl] 17α-hydroxy 17β-(3-hydroxy propyl) 13α-méthyl gona-4,9-dièn-3-one (Onapristone).

La présente invention a plus précisément pour objet l'application telle que définie précédemment, caractérisée en ce que les composés ayant une activité antiglucocorticoïde répondent à la formule générale (VIII) :

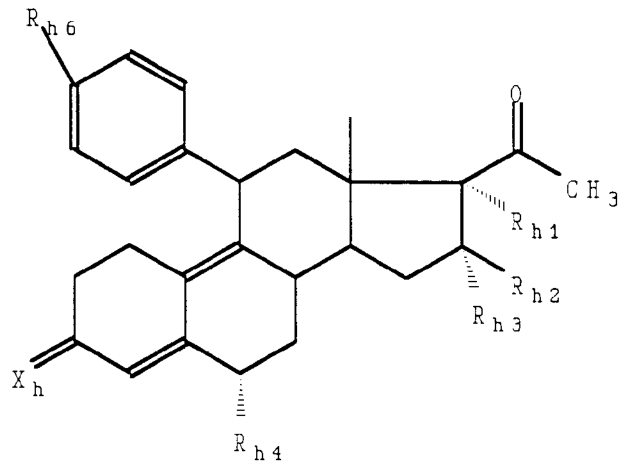

(VIII)

dans laquelle :

(i) $R_{h1}$ représente un atome d'hydrogène, un groupement alkyle, alkényle ou alkynyle ayant au plus 4 atomes de carbone, un groupement -OH, un groupement $OC(O)CH_3$ ou un groupement $OC(O)R_{h5}$ dans lequel $R_{h5}$ représente un groupement alkyle, alkényle ou alkynyle ayant chacun au plus 8 atomes de carbone, ou un groupement aryle, $R_{h2}$ représente un atome d'hydrogène, $R_{h3}$ représente un atome d'hydrogène, un groupement alkyle, alkényle ou alkynyle ayant chacun au plus 4 atomes de carbone, $R_{h4}$ représente un atome d'hydrogène, un groupement méthyle, fluor ou chlore, $R_{h6}$ représente un atome d'hydrogène ou un groupement $(CH_3)_2N$, $CH_3O$, $CH_3CO$, $CH_3S$, $CH_3SO$, $CH_3SO_2$ et $X_h$ représente O ou $NOCH_3$, ou

(ii) $R_{h1}$ et $R_{h2}$ forment ensemble une liaison carbone-carbone et $R_{h3}$, $R_{h4}$, $R_{h6}$ et $X_h$ sont tels que définis plus haut, ou

(iii) $R_{h1}$ et $R_{h3}$ forment ensemble un groupement $-CH_2-$ ou $-N=N-CH_2-$, $R_{h2}$ représente un atome d'hydrogène et $R_{h4}$, $R_{h6}$ et $X_h$ sont tels que définis précédemment, ou

(iv) $R_{h2}$ et $R_{h3}$ forment ensemble un groupement $=CH_2$ et $R_{h1}$, $R_{h4}$, $R_{h6}$ et $X_h$ sont tels que définis précédemment,

ainsi que leurs sels d'addition avec les acides pharmaceutiquement acceptables.

Les produits de formule générale (VIII) sont décrits et préparés dans la demande de brevet WO 89/12448.

Les définitions des divers substituants de ladite formule ainsi que les valeurs particulières desdits substituants et l'ensemble des produits synthétisés qui sont considérés comme des produits préférés dans le cadre de la présente invention sont définis dans la demande de brevet précitée.

La présente invention a tout spécialement pour objet l'application telle que définie précédemment du produit, répondant à la formule générale (VIII), suivant :

- 17α-acétyloxy 11β-[4-(diméthylamino) phényl] 19-nor-pregna 4,9-diène-3,20-dione.

La présente invention a plus précisément pour objet l'application telle que définie précédemment, caractérisée en ce que le composé ayant une activité antiglucocorticoïde est la :

- 11β-[4-(diméthylamino) phényl] 17β-hydroxy 17α-(3-hydroxy 1-propényl) estra-4,9-dièn-3-one (lilopristone).

Ce produit est décrit dans Research Disclosure RD286036 et RD276106.

L'invention s'étend aux compositions pharmaceutiques renfermant comme principe actif au moins un médicament tel que défini ci-dessus.

Les composés de l'invention sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend notamment aux compositions pharmaceutiques renfermant comme principe actif au moins un médicament tel que défini ci-dessus, caractérisées en ce que les composés répondent à l'une quelconque des formules générales (I), (II), (III), (IV), (V), (VI), (VII) ou (VIII) telles que définies précédemment.

L'invention s'étend plus particulièrement aux compositions pharmaceutiques renfermant comme principe

actif au moins un médicament tel que défini ci-dessus, caractérisées en ce que les composés répondant à l'une quelconque des formules générales (I), (II), (III), (IV), (V), (VI), (VII) ou (VIII) sont choisis dans la liste suivante :

- 17β-hydroxy 11β-(4-diméthylamino phényl) 17α-(prop-1-ynyl) estra 4,9-dièn-3-one (Mifépristone),
- N-oxyde du 21-chloro 17β-hydroxy 11β-(4-diméthylamino phényl) 17α-19-nor-pregna-4,9-dièn-20-yn-3-one,
- (Z) 11β-[4-(diméthylamino) phényl] 17β-hydroxy 17α-[(Z)-1-propényl] estra-4,9-dièn-3-one,
- 11β-[(4-méthylthio) phényl] 17β-hydroxy 17α-(prop-1-ynyl) estra-4,9-dièn-3-one,
- 9α,10α-époxy 17β-hydroxy 11β-[(4-méthylsulfonyl) phényl] 17α-(prop-1-ynyl) estr-4-èn-3-one,
- sel de sodium de l'acide [[4-[21-chloro 17β-hydroxy 3-oxo 19-nor 17α-pregna-4,9-dièn-20-yn-[11β-yl] phényl] méthylamino] acétique,
- sel de sodium de l'acide [[4-[17β-hydroxy 3-oxo 17α-(2-propényl) estra-4,9-dièn-11β-yl] phényl] méthylamino] acétique,
- [[4-[17β-hydroxy 3-oxo 17α-(1-propynyl) estra-4,9-dièn-11β-yl] phényl] méthylamino] acétate d'éthyle,
- sel de sodium de l'acide [[4-[17β-hydroxy 3-oxo 17α-(1-propynyl) estra-4,9-dièn-11β-yl] phényl] méthylamino] acétique,
- γ-lactone de l'acide 17β-hydroxy 11β-[4-(diméthylamino) phényl] 21-méthylène 3-oxo 19-nor 17α-pregna-4,9-diène-21-carboxylique,
- 17β-hydroxy 10β-[(4-méthyl phényl) méthyl] 17α-prop-1-ynyl) estra-4,9(11)dièn-3-one,
- 17-(3-hydroxy prop-1(Z)-ényl) 17β-hydroxy 11β,19-(4-méthylthio-0-phénylène) 4-androstèn-3-one,
- 17-(prop-1-inyl) 17β-hydroxy 11β,19-(4-diméthylamino-0-phénylène) 4-androstèn-3-one,
- 17-(3-hydroxy prop-1(Z)-ényl) 17β-hydroxy 11β,19-(4-diméthylamino-0-phénylène) 4-androstèn-3-one,
- 17-(prop-1-inyl) 17β-hydroxy 11β,19-(4-acétyl-0-phénylène) 4-androstèn-3-one,
- 9α,17β-dihydroxy 11β,19-[4-(3-pyridyl)-0-phénylène] 17-(prop-1-inyl) 4-androstèn-3-one,
- 10β, 11β-(5-diméthylamino-0-phénylènethio) 17β-hydroxy 17α-(3-hydroxyprop-1(Z)-ényl) estr-4-èn-3-one,
- 11β-[4-(diméthylamino) phényl] 17α-hydroxy 17β-(3-hydroxy propyl) 13α-méthyl gona-4,9-dièn-3-one (Onapristone),
- 17α-acétyloxy 11β-[4-(diméthylamino) phényl] 19-nor-pregna 4,9-diène-3,20-dione,
- 11β-[4-(diméthylamino) phényl] 17β-hydroxy 17α-(3-hydroxy 1-propényl) estra-4,9-dièn-3-one.

L'invention s'étend tout particulièrement aux compositions pharmaceutiques renfermant comme principe actif un médicament tel que défini ci-dessus, caractérisées en ce que le composé répondant à la formule générale (I) est la Mifépristone ou 17β-hydroxy 11β-(4-diméthylamino phényl) 17α-(prop-1-ynyl) estra 4,9-dièn-3-one.

La posologie utile varie en fonction du type de sevrage à prévenir ou à traiter et de la voie d'administration. Elle peut varier de 1 à 1000 mg par jour chez l'adulte par voie orale.

## ETUDE PHARMACOLOGIQUE:

### Evaluation du syndrome de sevrage précipité par la naloxone chez la souris.

1 - Matériel et méthode.

1.1 Animaux

Les expériences ont été réalisées chez des souris mâles Swiss normales ou surrénalectomisées (Charles River, France) d'un poids corporel compris entre 20 et 25 g. Durant les essais, les animaux ont eu libre accès à la nourriture et à l'eau de boisson. Le nombre d'animaux par groupe est de 10 à 13 souris.

1.2 Produits

Les antiglucocorticoïdes :

- AG1 (17β-hydroxy 11β-(4-diméthylaminophényl) 17α-(prop-1-ynyl) estra 4,9-dièn-3-one),
- AG2 (17β-hydroxy 10β-[(4-méthylphényl) méthyl] 17α-(prop-1-ynyl) estra 4,9-dièn-3-one),
- AG3 (γ-lactone de l'acide 17β-hydroxy 11β-(4-diméthylaminophényl) 21-méthylène 3-oxo 19-nor 17α-(pregna 4,9-dièn-21-carboxylique)
- AG4 10β-benzyl 17β-hydroxy 17α-(prop-1-ynyl) estra 4,9(11)-dièn-3-one
- AG5 11β-[4-(diméthylamino) phényl] 17α-hydroxy 17β-(3-hydroxypropyl) 13α-méthyl gona-4,9-dièn-3-one (onapristone)

- AG6 11β-[4-(diméthylamino) phényl] 17β-hydroxy 17α-(3-hydroxy-1-propényl) estra-4,9-dièn-3-one (lilopristone)
- AG7 17α-acétoxy 11β-[4-(diméthylamino) phényl] 19-nor pregna 4,9-dièn-3,20-dione
- et l'antiprogestérone AP1 (17R 11β-[4-(méthylthio) phényl] spiro (estra-4,9-dièn-17,2'(5'H) furan) 3-one décrit dans WO 87/05908 ex. 3)

ont été mis en suspension dans de la méthyl cellusose à 0,5 % et administrés par voie orale (p.o). La morphine a été mise en solution dans du sérum physiologique et administrée par voie sous-cutanée (s.c). Enfin, le chlorhydrate de naloxone a été solubilisé dans de l'eau distillée et injecté par voie intra-péritonéale.

Tous les produits ont été administrées à l'animal sous un volume de 25 ml/kg.

1.3 Modalités de traitement et doses administrées.

1.3.1 Traitement par la morphine : induction de l'état de dépendance morphinique.

Le premier jour (J1), les animaux ont reçu 5 injections sous-cutanées avec des doses croissantes de morphine réparties dans la matinée et sur le début de l'après midi, à une heure d'intervalle. Les 3 jours suivants (J2, J3, J4), ces mêmes animaux ont été traités le matin par deux administrations de la dose maximale utilisée au jour 1.

| Morphine - Doses (mg/kg, s.c.) | | | | Dose cumulée (mg/kg) |
|---|---|---|---|---|
| J1 | J2 | J3 | J4 | |
| 8<br>16<br>25<br>50<br>100 | 100<br>100 | 100<br>100 | 100<br>100 | 799 |

1.3.2 Traitement répété durant 4 jours.

Les antiglucocorticoïdes AG1, AG2, AG3, AG4 et AG5 ont été administrées à la dose de 100 mg/kg par voie orale sous forme d'un traitement quotidient effectué 2 heures avant l'administration de morphine et ceci durant 4 jours.

| JOUR 1 | JOUR 2 | JOUR 3 | JOUR 4 |
|---|---|---|---|
| Antiglucocorticoïde<br>--> voie orale | | | |
| ↓ 2 heures | idem | idem | idem |
| Morphine<br>8<br>16<br>25<br>50<br>100 mg/kg,sc | <br>100<br>100 | <br>100<br>100 | <br>100<br>100 |
| Dose cumulée<br>de 199 mg/kg | | | ↓ 3 heures<br>Sevrage naloxone<br>100 mg/kg,ip<br>↓<br>Comportement stéréotypé<br>--> Sauts |

1.3.3 Traitement aigü.

Le composé AG1 (20 et 100 mg/kg), le composé AG3 (100 mg/kg), le composé AG2 (100 mg/kg), le compoé AG4 (100 mg/kg), le composé AG5 (200 mg/kg), le composé AG6 (100 mg/kg), le composé AG7 (20 et 100 mg/kg) et l'antiprogestérone AP1 (100 mg/kg) ont été administrés sous forme d'un traitement aigü réalisé 24 heures (J3) avant le sevrage par le chlorhydrate de naloxone (J4).

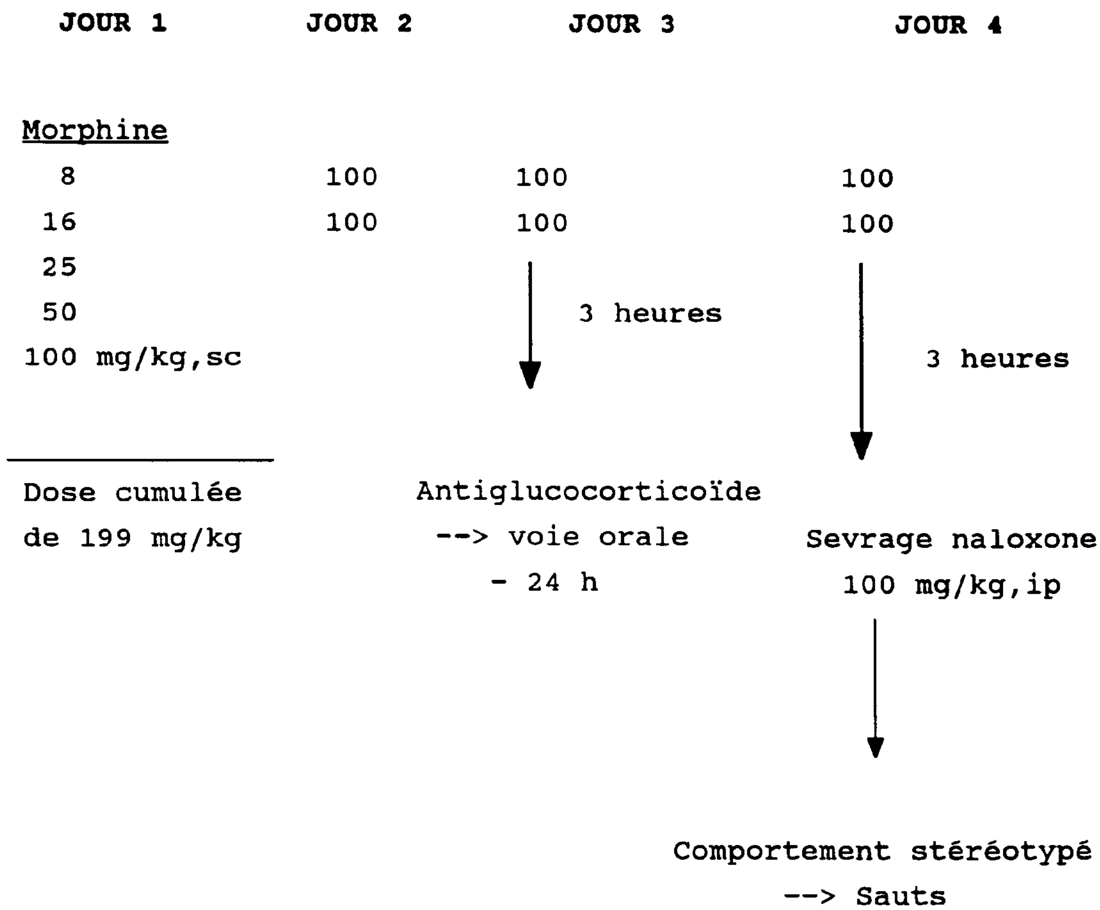


1.2 Mode opératoire

La technique utilisée s'inspire de celle décrite par Way et al. J. Pharmacol. Exp. Ther. (1969), 167 : 1-8 et Huidobro et Maggiolo Acta Physiol. Pharmacol. Latinoam. (1961), 11 : 201-9. Ainsi, l'administration réitérée d'un morphinique induit un phénomène de dépendance physique qui peut être facilement mis en évidence par la précipitation d'un syndrome de sevrage à l'aide d'un antagoniste opiacé tel que la naloxone. Ce syndrome se manifeste, chez la souris sous la forme d'un comportement stéréotypé caractérisé par l'apparition de sauts répétitifs dont le nombre est en relation avec la durée et l'intensité du traitement par le morphinique.

Le sevrage des animaux a été réalisé le quatrième jour; trois heures après le dernier traitement, à l'aide de chlorhydrate de naloxone injecté par voie intrapéritonéale à la dose de 100 mg/kg. Immédiatement après cette injection, les animaux ont été placés individuellement dans des cylindres en plexiglass (hauteur = 40 cm, diamètre = 20 cm). Le nombre de sauts consécutifs au sevrage et effectués par chaque animal a été compté durant une période de 10 minutes.

Les résultats qui figurent dans les tableaux représentent les moyennes des valeurs individuelles assorties de l'erreur standard à la moyenne (m ± esm).

2. Résultats.

2.1 Sevrage précipité par la naloxone chez des souris normales traitées par la morphine.

2.1.1 Traitement répété durant 4 jours.

| | Doses (mg/kg) | Nombre de sauts/10 minutes (m ± esm) | % protection |
|---|---|---|---|
| Témoins | 0 | 0 | |
| Morphine | 799 s.c (cumulée sur 4 jours) | 115 ± 21 | |
| AG1 | 4 x 100 p.o | 0 | |
| Morphine + AG1 | 799 s.c + 4 x 100 p.o. | 68 ± 12 * | - 41 |

| | Doses (mg/kg) | Nombre de sauts/10 minutes (m ± esm) | % protection |
|---|---|---|---|
| Témoins | 0 | 0 | |
| Morphine | 799 s.c (cumulée sur 4 jours) | 117 ± 14 | |
| AG2 | 4 x 100 p.o | 0 | |
| Morphine + AG2 | 799 s.c + 4 x 100 p.o | 79 ± 9 * | - 32 |
| AG3 | 4 x 100 p.o | 3 ± 2 | |
| Morphine + AG3 | 799 s.c + 4 x 100 p.o | 66 ± 7 ** | - 44 |

| | Doses (mg/kg) | Nombre de sauts/10 minutes (m ± esm) | % protection |
|---|---|---|---|
| Témoins | 0 | 0 | |
| Morphine | 799 s.c (cumulée sur 4 jours) | 103 ± 10 | |
| AG4 | 4 x 100 p.o | 1 ± 1 | |
| Morphine + AG4 | 799 s.c + 4 x 100 p.o | 72 ± 7 * | - 30 |
| Témoins | 0 | 4 ± 4 | |
| Morphine | 799 s.c (cumulée sur 4 jours) | 97 ± 13 | |
| AG5 | 4 x 100 p.o | 1 ± 1 | |
| Morphine + AG5 | 799 s.c + 4 x 100 p.o | 68 ± 12 * | - 30 |

2.1.2 Traitement aigü.

| | Doses (mg/kg) | Nombre de sauts/10 minutes (m ± esm) | % protection |
|---|---|---|---|
| Témoins | 0 | 5 ± 4 | |
| Morphine | 799 s.c (cumulée sur 4 jours) | 97 ± 11 | |
| AG1 | 100 p.o | 1 ± 1 | |
| Morphine + AG1 | 799 s.c + 100 p.o | 56 ± 10 ** | - 42 |
| Témoins | 0 | 9 ± 5 | |
| Morphine | 799 s.c (cumulée sur 4 jours) | 162 ± 15 | |
| AG1 | 20 p.o | 5 ± 3 | |
| Morphine + AG1 | 799 s.c + 20 p.o | 115 ± 16 * | - 29 |

| | Doses (mg/kg) | Nombre de sauts/10 minutes (m ± esm) | % protection |
|---|---|---|---|
| Témoins | 0 | 0 | |
| Morphine | 799 s.c (cumulée sur 4 jours) | 100 ± 12 | |
| AG2 | 100 p.o | 2 ± 1 | |
| Morphine + AG2 | 799 s.c + 100 p.o | 100 ± 13 NS | |
| AG3 | 100 p.o | 4 ± 2 | |
| Morphine + AG3 | 799 s.c + 100 p.o | 68 ± 10 * | - 32 |
| Témoins | 0 | 7 ± 3 | |
| Morphine | 799 s.c (cumulée sur 4 jours) | 137 ± 13 | |
| AP1 | 100 p.o | 6 ± 2 | |
| Morphine + AP1 | 799 s.c + 100 p.o | 116 ± 7 NS | - 15 |

| | Doses (mg/kg) | Nombre de sauts/10 minutes (m ± esm) | % protection |
|---|---|---|---|
| Témoins | 0 | 4 ± 3 | |
| Morphine | 799 s.c (cumulée sur 4 jours) | 109 ± 14 | |
| AG4 | 100 p.o | 0 ± 0 | |
| Morphine + AG4 | 799 s.c + 100 p.o | 96 ± 13 NS | - 12 |
| Témoins | 0 | 2 ± 2 | |
| Morphine | 799 s.c (cumulée sur 4 jours) | 93 ± 11 | |
| AG5 | 100 p.o | 3 ± 3 | |
| Morphine + AG5 | 799 s.c + 100 p.o | 74 ± 10 NS | - 20 |
| Témoins | 0 | 5 ± 5 | |
| Morphine | 799 s.c (cumulée sur 4 jours) | 97 ± 10 | |
| AG6 | 100 p.o | 11 ± 3 | |
| Morphine + AG6 | 799 s.c + 100 p.o | 80 ± 9 NS | - 17 |

| | Doses (mg/kg) | Nombre de sauts/10 minutes (m ± esm) | % protection |
|---|---|---|---|
| Témoins | 0 | 2 ± 2 | |
| Morphine | 799 s.c (cumulée sur 4 jours) | 106 ± 10 | |
| AG7 | 100 p.o | 7 ± 4 | |
| Morphine + AG7 | 799 s.c + 100 p.o | 45 ± 5 ** | - 58 |
| Témoins | 0 | 2 ± 1 | |
| Morphine | 799 s.c (cumulée sur 4 jours) | 101 ± 11 | |
| AG7 | 20 p.o | 2 ± 1 | |
| Morphine + AG7 | 799 s.c + 10 p.o | 74 ± 10 * | - 26 |

* p. <0,05 ; ** p. <0,01 selon le test de Dunnett par rapport au groupe morphine, n = 10 souris/groupe.

2.2 a) Comparaison du syndrome de sevrage précipité par la naloxone chez la souris normale ou surrenalectomisée traitée par la morphine (799 mg/kg, s.c, dose cumulée sur 4 jours).

| | Nombre de sauts/10 minutes (m ± ems) |
|---|---|
| Souris normales | 103 ± 12 |
| Souris surrénalectomisées | 72 ± 7 * (-30 %) |

2.2 b) Comparaison du syndrome de sevrage précipité par la naloxone chez la souris normale ou surrenalectomisée traitée par la morphine (399 mg/kg, s.c, dose cumulée sur 4 jours).

| | Nombre de sauts/10 minutes (m ± ems) |
|---|---|
| Souris normales | 87 ± 12 |
| Souris surrénalectomisées | 49 ± 3 ** (-43 %) |

* p. <0,05 selon le test de Dunnett, n = 17-20 souris/groupe (regroupement de deux expériences).

** p. <0,01 selon le test de Dunnett, n = 10 souris/groupe.

Discussion - Conclusion

Co-administré durant quatre jours avec la morphine, le composé AG1 (100 mg/kg, p.o) inhibe de façon significative (-41 %) le syndrome de sevrage précipité par la naloxone chez des souris dépendantes à la morphine. Cet effet est observé 25 de façon similaire avec le composé AG3 (-44 %) et dans une moindre mesure avec le composé AG2 (-32 %) lorsque ces produits ont été administrés à la dose de 100 mg/kg, p.o dans les mêmes conditions expérimentales que le composé AG1.

Administré sous la forme d'un traitement unique effectué 30 24 heures avant le sevrage précipité par la naloxone, le composé AG1 (100 mg/kg) s'oppose de façon significative (-42 %) et dans des proportions comparables à celles d'un traitement répété, au sevrage morphinique. Ce produit manifeste une activité encore non négligeable à la plus 35 faible dose de 20 mg/kg, p.o (-29 %). L'administration unique, du composé AG3 (100 mg/kg, p.o) se traduit par une inhibition moins importante du syndrome de sevrage (-32 %) alors que celle du composé AG2 (100 mg/kg,p.o) se révèle inactive dans ces conditions de traitement.

Le composé AG1 étant pourvu, comme le composé AG3, d'une activité anti-progestérone, il a été recherché la participation éventuelle de cette activité dans la prévention du syndrome de sevrage morphinique précipité par la naloxone. Pour ce faire, l'anti-progestérone dissociée AP1 (Philibert et al. 1989, New analogue of mifépristone with more dissociated antiprogestérone activities, J. Steroid Biochem. 34, 413) a été étudié, et administré sous forme d'un traitement aigü, 24 heures avant le sevrage par la naloxone, à la dose de 100 mg/kg, p.o. Ce composé n'a pas modifié de façon significative le syndrome de sevrage morphinique.

Enfin un autre élément pour la participation probable des glucocorticoïdes endogènes dans le phénomène de sevrage opiacé était obtenu chez la souris surrenalectomisée traitée à la morphine aux doses cumulées de 799 mg s.c. ou de 399 mg s.c., chez laquelle le syndrome de sevrage précipité par la naloxone était plus atténué (-30 %) et (-43%) respectivement que chez la souris normale.

En conclusion, ces résultats confirment la participation des glucocorticoïdes endogènes dans le syndrome de sevrage morphinique, tel qu'il se manifeste chez la souris par un comportement stéréotypé de sauts, puisque ce dernier est inhibé par des antiglucocorticoïdes ou par surrenalectomie.

Le composé AG1 se révèle comme étant le produit le plus actif parmi les trois molécules antiglucocorticoïdes testées.

L'absence de différence entre les traitements répétés et aigüs dans l'activité du composé AG1 suggère que ce composé pourrait agir préférentiellement au moment de la phase de sevrage.

L'activité des antiglucocorticoïdes, à travers les résultats obtenus avec les composés AG1, AG3 et AG2 montre qu'un antagoniste des glucocorticoïdes peut avoir un effet bénéfique en clinique humaine dans la prévention et traitement du syndrome de sevrage narcotique.

## Revendications

**1)** Application des composés ayant une activité antiglucocorticoïde pour la préparation de médicaments destinés à la prévention ou au traitement des manifestations liées au syndrome de sevrage spontané ou précipité des narcotiques ou mélanges de narcotiques.

**2)** Application selon la revendication 1, caractérisée en ce que le narcotique est un morphinomimétique choisi parmi l'héroïne, la morphine et la méthadone.

**3)** Application selon la revendication 1, caractérisée en ce que le narcotique est la cocaïne.

**4)** Application selon l'une quelconque des revendications 1 à 3, caractérisée en ce que les composés ayant une activité antiglucocorticoïde répondent à la formule générale (I) :

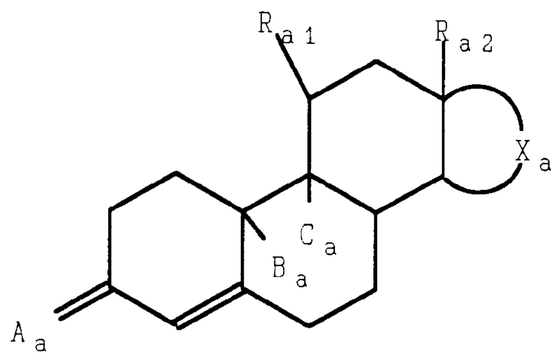

(I)

dans laquelle $R_{a1}$ représente un radical organique renfermant de 1 à 18 atomes de carbone, contenant au moins un atome d'azote, de phosphore ou de silicium, l'atome immédiatement adjacent au carbone en 11 étant un atome de carbone, $R_{a2}$ représente un radical hydrocarboné renfermant de 1 à 8 atomes de carbone, $X_a$ représente le reste d'un cycle pentagonal ou hexagonal éventuellement substitué et éventuellement porteur d'insaturation, le groupement $C = A_a$ en position 3 représente un groupement oxo, libre ou bloqué sous forme de cétal, un groupement :

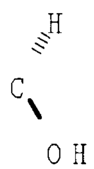 , 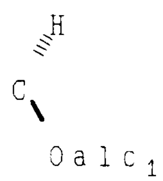 , 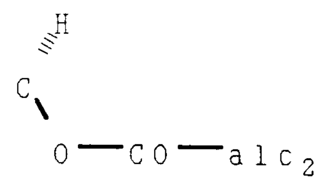 ,

un groupement C = NOH, un groupement $C{=}NO\text{-}alc_3$, ou un groupement $CH_2$, $alc_1$, $alc_2$ et $alc_3$ représentant un radical alkyle renfermant de 1 à 8 atomes de carbone ou un groupement aralkyle renfermant de 7 à 15 atomes de carbone et $B_a$ et $C_a$ forment ensemble une double liaison ou un pont époxyde, ainsi que leurs sels d'addition avec les acides pharmaceutiquement acceptables.

**5)** Application selon la revendication 4, caractérisée en ce que les composés répondant à la formule générale (I) sont choisis dans la liste suivante :

- 17β-hydroxy 11β-(4-diméthylamino phényl) 17α-(prop-1-ynyl) estra 4,9-dièn-3-one (Mifépristone),
- N-oxyde du 21-chloro 17β-hydroxy 11β-(4-diméthylamino phényl) 17α-l9-nor-pregna-4,9-dièn-20-yn-3-one,
- (Z) 11β-[4-(diméthylamino) phényl] 17β-hydroxy 17α-[(Z)-1-propényl] estra-4,9-dièn-3-one,

ainsi que leurs sels d'addition avec les acides pharmaceutiquement acceptables.

**6)** Application selon la revendication 4, caractérisée en ce que le composé répondant à la formule générale (I) est la :

- 17β-hydroxy 11β-(4-diméthylamino phényl) 17α-(prop-1-ynyl) estra 4,9-dièn-3-one (Mifépristone).

**7)** Application selon l'une quelconque des revendications 1 à 3, caractérisée en ce que les composés ayant une activité antiglucocorticoïde répondent à la formule générale (II) :

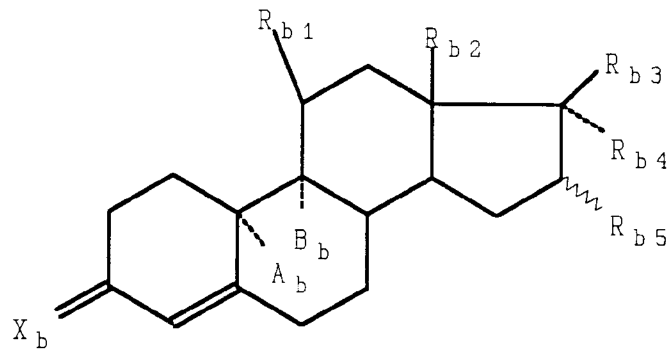

(II)

dans laquelle soit $R_{b1}$ représente un radical thiényle éventuellement substitué, un radical furyle, un radical cycloalkyle ayant de 3 à 6 atomes de carbone, un radical phényle éventuellement substitué par un ou plusieurs radicaux choisis parmi les radicaux hydroxy, halogène, trifluorométhyle, alkyle, alkoxy, alkylthio éventuellement oxydé sous forme de sulfoxyde ou de sulfone, alkényloxy ayant au plus 6 atomes de carbone et phényloxy, soit $R_{b1}$ représente un radical naphtyle ou phényl-phényle ou un radical alkyle ou alkényle portant éventuellement plusieurs insaturations et ayant au plus 6 atomes de carbone,
$R_{b2}$ représente un radical méthyle ou éthyle,
$R_{b3}$ représente un atome d'hydrogène, un radical alkyle, alkényle ou alkynyle éventuellement substitué, un radical hydroxy, acétyle, hydroxyacétyle, carboxyalkoxy ayant de 2 à 4 atomes de carbone éventuellement estérifié ou salifié, hydroxy-alkyle éventuellement estérifié,
$R_{b4}$ représente un atome d'hydrogène, un radical hydroxy ou un radical alkyle, alkényle ou alkynyle ayant au plus 12 atomes de carbone éventuellement substitué par un radical amino, alkylamino, dialkylamino, ou par un halogène, un radical alkylthio, alkoxy, trialkylsilyl ou cyano,
$R_{b5}$ représente un atome d'hydrogène ou un radical méthyle en position $\alpha$ ou $\beta$,
$X_b$ représente un atome d'oxygène ou un radical hydroxyimino ou alkoxyimino ayant de 1 à 4 atomes de carbone, en position syn ou anti ;
$A_b$ et $B_b$ représentent une fonction a-époxy ou une seconde liaison entre les carbones 9 et 10 ;
ainsi que leurs sels d'addition avec les acides pharmaceutiquement acceptables lorsque $R_{b4}$ représente un radical comportant une fonction amino.

**8)** Application selon la revendication 7, caractérisée en ce que les composés répondant à la formule générale (II) sont choisis dans la liste suivante :

- 11β-[(4-méthylthio) phényl] 17β-hydroxy 17α-(prop-1-ynyl) estra-4,9-dièn-3-one,
- 9α,10α-époxy 17β-hydroxy 11β-[(4-méthylsulfonyl) phényl] 17α-(prop-1-ynyl) estr-4-èn-3-one.

**9)** Application selon l'une quelconque des revendications 1 à 3, caractérisée en ce que les composés ayant une activité antiglucocorticoïde répondent à la formule générale (III) :

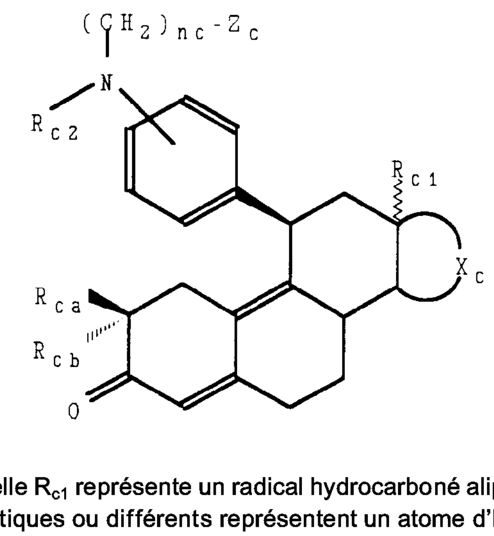

(III)

dans laquelle $R_{c1}$ représente un radical hydrocarboné aliphatique renfermant de 1 à 8 atomes de carbone, $R_{ca}$ et $R_{cb}$ identiques ou différents représentent un atome d'hydrogène, un radical alkyle renfermant de 1 à 4 atomes de carbone, $R_{c2}$ représente un atome d'hydrogène ou un radical alkyle renfermant de 1 à 12 atomes de carbone et éventuellement substitué, nc représente un entier de 1 à 6, $Z_c$ représente un groupe carboxy libre

ou salifié sous forme d'un sel de métal alcalin ou alcalino-terreux, d'un sel d'ammonium ou d'un sel d'amine, $X_c$ représente le reste d'un cycle pentagonal ou hexagonal éventuellement substitué et éventuellement porteur d'une insaturation, et le trait ondulé en position 13 signifie que $R_{c1}$ peut se trouver en position alpha ou béta.

**10)** Application selon la revendication 9, caractérisée en ce que les composés répondant à la formule générale (III) sont choisis dans la liste suivante :

- sel de sodium de l'acide [[4-[21-chloro 17β-hydroxy 3-oxo 19-nor 17α-pregna-4,9-dièn-20-yn-[11β-yl] phényl] méthylamino] acétique,
- sel de sodium de l'acide [[4-[17β-hydroxy 3-oxo 17α-(2-propényl) estra-4,9-dièn-11β-yl] phényl] méthylamino] acétique,
- [[4-[17β-hydroxy 3-oxo 17α-(1-propynyl) estra-4,9-dièn-11β-yl] phényl] méthylamino] acétate d'éthyle,
- sel de sodium de l'acide [[4-[17β-hydroxy 3-oxo 17α-(1-propynyl) estra-4,9-dièn-11β-yl] phényl] méthylamino] acétique.

**11)** Application selon l'une quelconque des revendications 1 à 3, caractérisée en ce que les composés ayant une activité antiglucocorticoïde répondent à la formule générale (IV) :

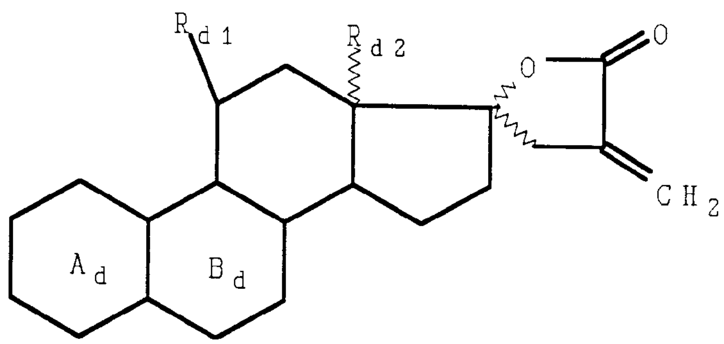

(IV)

dans laquelle $R_{d1}$ représente :

soit un radical phényle, biphényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle, chacun de ces radicaux étant éventuellement substitué par un ou plusieurs radicaux choisis parmi :

- les radicaux alkyle, alkényle ou alkynyle ayant au plus 8 atomes de carbone et éventuellement substitués par un ou plusieurs radicaux choisis parmi les radicaux hydroxyle, halogène, oxo, trialkylsilyle, alkoxy ou alkylthio, les radicaux alkyle, alkoxy et alkylthio ayant de 1 à 4 atomes de carbone,
- les radicaux alkoxy, alkényloxy ou alkylthio éventuellement oxydés sous forme de sulfoxyde ou de sulfone, tous ces radicaux ayant au plus 4 atomes de carbone,
- les atomes d'halogène,
- les radicaux trialkylsilyle, chaque radical alkyle comportant de 1 à 4 atomes de carbone,
- les radicaux hydroxyle, trifluorométhyle, acyle ayant de 1 à 6 atomes de carbone, carboxy libre estérifié ou salifié,
- le radical

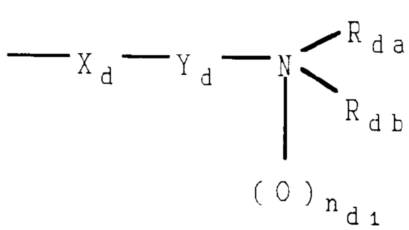

dans lequel $X_d$ représente une simple liaison, un atome d'oxygène, de soufre ou un radical

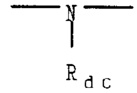

$Y_d$ représente une simple liaison, un radical alkylène, alkénylène ou alkynylène linéaire ou ramifié ayant au plus 8 atomes de carbone, $n_{d1}$ représente 0 ou 1,

$R_{da}$ et $R_{db}$ identiques ou différents représentent :

. un atome d'hydrogène,

. un radical alkyle ayant de 1 à 4 atomes de carbone éventuellement substitué par un radical carboxy libre estérifié ou salifié, un radical trialkylsilyle, chaque radical alkyle comportant de 1 à 4 atomes de carbone,

. un radical acyle ayant de 1 à 8 atomes de carbone,

ou $R_{da}$ et $R_{db}$ forment avec l'atome de carbone auquel ils sont liés un hétérocycle comportant éventuellement un autre hétéroatome choisi parmi l'oxygène, le soufre ou l'azote et éventuellement substitué par un radical alkyle ayant de 1 à 4 atomes de carbone, étant entendu que lorsque $X_d$ représente un atome de soufre, d'oxygène ou un radical

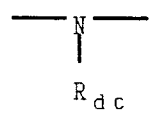

$Y_d$ ne peut pas être une simple liaison ou un radical méthylène,

$R_{dc}$ représente un atome d'hydrogène ou un radical alkyle ayant de 1 à 4 atomes de carbone,

soit $R_{d1}$ représente un radical indényle ou quinoléinyle éventuellement substitué par un radical alkyle ayant de 1 à 4 atomes de carbone et éventuellement hydrogéné,

$R_{d2}$ en position $\alpha$ ou $\beta$ représente un radical alkyle ayant de 1 à 4 atomes de carbone,

les traits ondulés en position 17 indiquent que le cycle lactonique peut se trouver dans l'une ou l'autre des positions possibles,

les cycles $A_d$ et $B_d$ représentent l'une de deux structures suivantes :

a) soit $A_d$ et $B_d$ représentent le groupement :

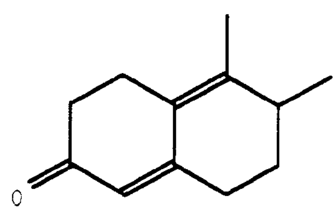

b) soit $A_d$ et $B_d$ représentent le groupement :

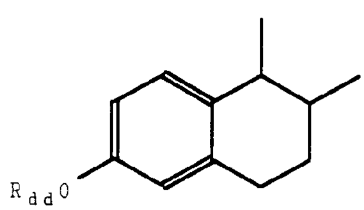

dans lequel $R_{dd}$ représente un atome d'hydrogène ou un radical alkyle ayant de 1 à 4 atomes de carbone éventuellement substitué par un ou plusieurs radicaux choisis parmi les radicaux hydroxyle et les atomes d'halogènes, ou $R_{dd}$ représente un radical arylalkyle ayant au plus 12 atomes de carbone ou un radical acyle ayant de 1 à 6 atomes de carbone,

ainsi que les sels des produits de formule (IV) avec les acides et les bases pharmaceutiquement acceptables.

**12)** Application selon la revendication 11, caractérisée en ce que le composé répondant à la formule générale (IV) est la :

- γ-lactone de l'acide 17β-hydroxy 11β-[4-(diméthylamino) phényl] 21-méthylène 3-oxo 19-nor 17α-pregna-4,9-diène-21-carboxylique.

**13)** Application selon l'une quelconque des revendications 1 à 3, caractérisée en ce que les composés ayant une activité antiglucocorticoïde répondent à la formule générale (V)

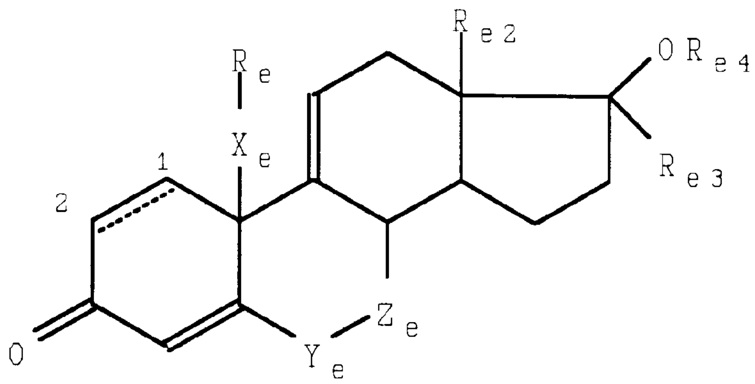

(V)

dans laquelle $X_e$ et $R_e$ sont tels que :

- soit $X_e$ représente un radical méthylène et $R_e$ représente
  ou bien un radical aryle carbocyclique ou hétérocyclique éventuellement substitué,
  ou bien un radical vinyle ou éthynyle éventuellement substitué,
- soit $X_e$ représente un atome de soufre ou une simple liaison et $R_e$ représente un radical aryle carbocyclique ou hétérocyclique éventuellement substitué,

$R_{e2}$ représente un radical méthyle ou éthyle,
$R_{e3}$ représente un radical alkyle, alkényle ou alkynyle ayant au plus 8 atomes de carbone, éventuellement substitué,
$R_{e4}$ représente un atome d'hydrogène ou un radical acyle,

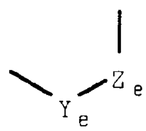

représente :
ou bien un groupement

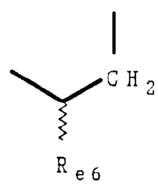

dans lequel $R_{e6}$ représente un atome d'hydrogène ou un radical méthyle en position α et/ou β,
ou bien un groupement

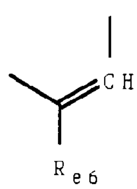

dans lequel $R_{e6}$ représente un atome d'hydrogène ou un radical méthyle,
ou bien un groupement

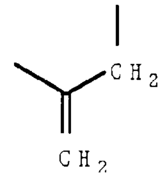

le trait pointillé en position 1(2) indique la présence éventuelle d'une seconde liaison carbone-carbone.

**14)** Application selon la revendication 13, caractérisée en ce que le composé répondant à la formule générale (V) est la :

- 17β-hydroxy 10β-[(4-méthyl phényl) méthyl] 17α-prop-1-ynyl) estra-4,9(11)dièn-3-one,
- 10β-benzyl 17β-hydroxy 17α-(prop-1-ynyl) estra 4,9(11)-dièn-3-one.

**15)** Application selon l'une quelconque des revendications 1 à 3, caractérisée en ce que les composés ayant une activité antiglucocorticoïde répondent à la formule générale (VI)

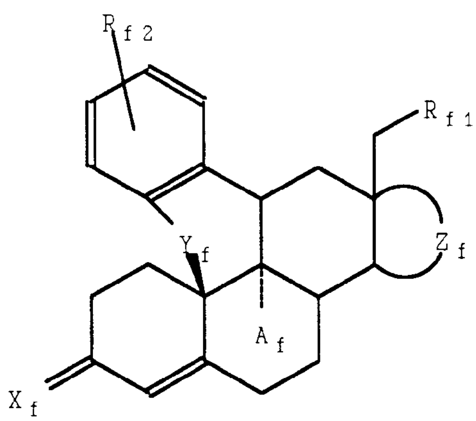

(VI)

dans laquelle :

$R_{f1}$ représente un atome d'hydrogène ou un groupement méthyle,

$R_{f2}$ en position 4 ou 5 représente un atome d'hydrogène, un groupement cyano, un groupement hétéroaryle, un groupement alkyle, alkényle ou alkynyle ayant jusqu'à 20 atomes de carbone éventuellement substitué par un ou plusieurs groupement oxo, un groupement $OR_{f3}$, un groupement $SR_{f3}$ dans lesquels $R_{f3}$ représente un atome d'hydrogène ou un groupement alkyle ayant de 1 à 8 atomes de carbone, un groupement $OSO_2R_{f11}$ dans lequel $R_{f11}$ représente un groupement alkyle ayant de 1 à 4 atomes de carbone perfluoré ou un groupement $NR_{f3}R_{f4}$ dans lequel

soit $R_{f3}$ est tel que défini plus haut et $R_{f4}$ identique ou différent de $R_{f3}$ représente un atome d'hydrogène, un groupement alkyle ayant de 1 à 10 atomes de carbone, un groupement cyano ou un groupement acyle ayant de 2 à 10 atomes de carbone,

soit $R_{f3}$ et $R_{f4}$ forment ensemble avec l'azote auquel ils sont liés un hétérocycle à 5 ou 6 chaînons comportant éventuellement un second hétéroatome choisi parmi N, O et S,

$X_f$ représente un groupement céto ou oxime,

$Z_f$ représente le reste d'un cycle pentagonal ou hexagonal éventuellement substitué et éventuellement porteur d'insaturations,

$Y_f$ représente un groupement $-CH_2-$ ou un atome de soufre et

$A_f$ représente un atome d'hydrogène ou représente un groupement OH dans le cas où $Y_f$ n'est pas un atome de soufre, ainsi que les sels d'addition avec les acides ou les bases pharmaceutiquement acceptables.

**16)** Application selon la revendication 15, caractérisée en ce que les composés répondant à la formule générale (VI) sont choisis dans la liste suivante :

- 17-(3-hydroxy prop-1(Z)-ényl) 17β-hydroxy 11β,19-(4-méthylthio-0-phénylène) 4-androstèn-3-one,
- 17-(prop-1-inyl) 17β-hydroxy 11β,19-(4-diméthylamino-0-phénylène) 4-androstèn-3-one,
- 17-(3-hydroxy prop-1(Z)-ényl) 17β-hydroxy 11β,19-(4-diméthylamino-0-phénylène) 4-androstèn-3-one,
- 17-(prop-1-inyl) 17β-hydroxy 11β,19-(4-acétyl-0-phénylène) 4-androstèn-3-one,
- 9α,17β-dihydroxy 11β,19-[4-(3-pyridyl)-0-phénylène] 17-(prop-1-inyl) 4-androstèn-3-one,
- 10β,11β-(5-diméthylamino-0-phénylènethio) 17β-hydroxy 17α-(3-hydroxyprop-1(Z)-ényl) estr-4-èn-3-one.

**17)** Application selon l'une quelconque des revendications 1 à 3, caractérisée en ce que les composés ayant une activité antiglucocorticoïde répondent à la formule générale (VII) :

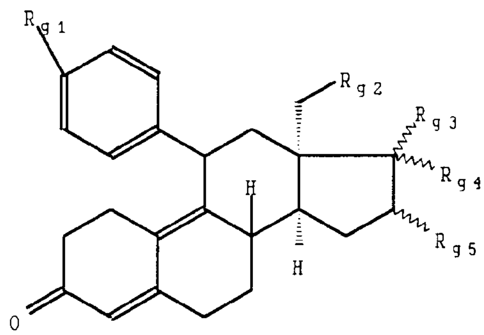

(VII)

dans laquelle :

$R_{g1}$ représente ou bien le groupement $NR_{gI}R_{gII}$ éventuellement oxydé sous forme de N-oxyde, dans lequel soit $R_{gI}$ et $R_{gII}$ identiques ou différents représentent un atome d'hydrogène ou un groupement alkyle ayant de 1 à 4 atomes de carbone, soit $R_{gI}$ et $R_{gII}$ forment ensemble avec l'atome d'azote auquel ils sont liés un hétérocycle à 5 ou 6 chaînons comportant éventuellement un second hétéroatome choisi parmi N, O ou S, ou bien le groupement $OR_{gIII}$ dans lequel $R_{gIII}$ représente un groupement méthyle, éthyle, propyle, méthoxyphényle, alkyle ou β-diméthylamino éthyle,

$R_{g2}$ représente un atome d'hydrogène, un groupement méthyle ou éthyle,

soit $R_{g3}$ représente un groupement $-(CH_2)_{ng}-CH_3$ dans lequel ng est égal à 0 à 4, un groupement $-(CH_2)_{ng}-CH_2-O-R_{gIV}$ ou $-(CH_2)_{ng}-CH_2-S-R_{gIV}$ dans lequel ng est égal à 0 à 4 et $R_{gIV}$ représente un atome d'hydrogène ou un groupement alkyle ayant de 1 à 4 atomes de carbone, un groupement $-CH=CH-(CH_2)_{ng}-OR_{gV}$, dans lequel ng est égal à 0 à 4 et $R_{gV}$ représente un atome d'hydrogène, un groupement alkyle ou alkanoyle ayant chacun de 1 à 4 atomes de carbone, un groupement $C{\equiv}C-X_g$, dans lequel $X_g$ représente un atome d'hydrogène, un groupement alkyle ayant de 1 à 4 atomes de carbone ou un halogène, un groupement $(CH_2)_{ng}-CH_2CN$ dans lequel ng est égal à O à 3 ou un groupement $-CO-CH_2-Y_g$ dans lequel $Y_g$ représente un atome d'hydrogène ou $OR_{gV}$, $R_{gV}$ étant tel que défini précédemment, et $R_{g4}$ représente un groupement hydroxy, alkoxy ou alkényloxy ayant chacun de 1 à 4 atomes de carbone,

soit $R_{g3}$ et $R_{g4}$ forment ensemble le cycle

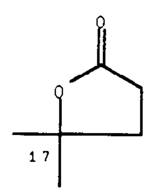

avec $R_{g3}$ en position α et $R_{g4}$ en position β ou $R_{g3}$ en position β et $R_{g4}$ en position α

et $R_{g5}$ représente un atome d'hydrogène ou un groupement alkyle ayant de 1 à 4 atomes de carbone en position α ou β, ainsi que leurs sels d'addition avec les acides et les bases pharmaceutiquement acceptables.

**18)** Application selon la revendication 17, caractérisée en ce que le composé répondant à la formule générale (VII) est la :

- 11β-[4-(diméthylamino) phényl] 17α-hydroxy 17β-(3-hydroxy propyl) 13α-méthyl gona-4,9-dièn-3-one (Onapristone).

**19)** Application selon l'une quelconque des revendications 1 à 3, caractérisée en ce que les composés ayant une activité antiglucocorticoïde répondent à la formule générale (VIII) :

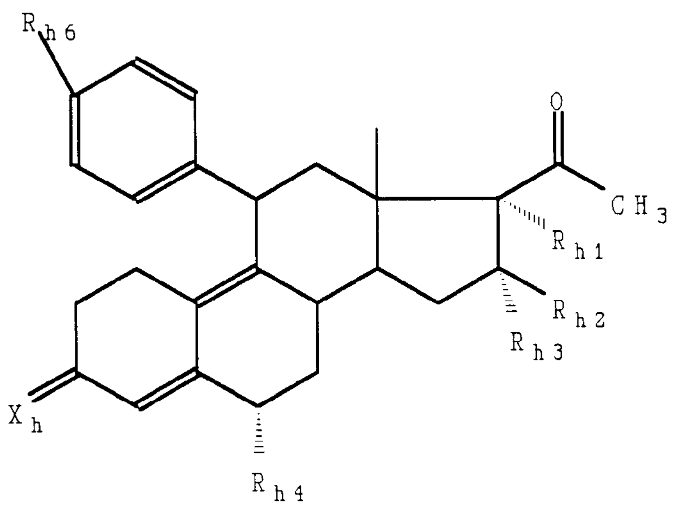

(VIII)

dans laquelle :

(i) $R_{h1}$ représente un atome d'hydrogène, un groupement alkyle, alkényle ou alkynyle ayant au plus 4 atomes de carbone, un groupement -OH, un groupement $OC(O)CH_3$ ou un groupement $OC(O)R_{h5}$ dans lequel $R_{h5}$ représente un groupement alkyle, alkényle ou alkynyle ayant chacun au plus 8 atomes de carbone, ou un groupement aryle, $R_{h2}$ représente un atome d'hydrogène, $R_{h3}$ représente un atome d'hydrogène, un groupement alkyle, alkényle ou alkynyle ayant chacun au plus 4 atomes de carbone, $R_{h4}$ représente un atome d'hydrogène, un groupement méthyle, fluor ou chlore, $R_{h6}$ représente un atome d'hydrogène ou un groupement $(CH_3)_2N$, $CH_3O$, $CH_3CO$, $CH_3S$, $CH_3SO$, $CH_3SO_2$ et $X_h$ représente O ou $NOCH_3$, ou

(ii) $R_{h1}$ et $R_{h2}$ forment ensemble une liaison carbone-carbone et $R_{h3}$, $R_{h4}$, $R_{h6}$ et $X_h$ sont tels que définis plus haut, ou

(iii) $R_{h1}$ et $R_{h3}$ forment ensemble un groupement $-CH_2-$ ou $-N=N-CH_2-$, $R_{h2}$ représente un atome d'hydrogène et $R_{h4}$, $R_{h6}$ et $X_h$ sont tels que définis précédemment, ou

(iv) $R_{h2}$ et $R_{h3}$ forment ensemble un groupement $=CH_2$ et $R_{h1}$, $R_{h4}$, $R_{h6}$ et $X_h$ sont tels que définis précédemment, ainsi que leurs sels d'addition avec les acides pharmaceutiquement acceptables.

**20)** Application selon la revendication 19, caractérisée en ce que le composé répondant à la formule générale (VIII) est la

- 17α-acétyloxy 11β-[4-(diméthylamino) phényl] 19-nor-pregna 4,9-diène-3,20-dione.

**21)** Application selon l'une quelconque des revendications 1 à 3, caractérisée en ce que le composé ayant une activité antiglucocorticoïde est la lilopristone ou

- 11β-[4-(diméthylamino) phényl] 17β-hydroxy 17α-(3-hydroxy 1-propényl) estra-4,9-dièn-3-one.

**22)** Les compositions pharmaceutiques, renfermant à titre de principe actif au moins un médicament tel que défini à la revendication 1, destinées à la prévention ou au traitement des manifestations liées au syndrome de sevrage spontané ou précipité des narcotiques ou mélanges de narcotiques.

**23)** Les compositions pharmaceutiques, renfermant à titre de principe actif au moins un médicament tel que défini à l'une quelconque des revendications 2 ou 3, destinées à la prévention ou au traitement des manifestations liées au syndrome de sevrage spontané ou précipité des narcotiques ou mélanges de narcotiques.

**24)** Les compositions pharmaceutiques, renfermant à titre de principe actif au moins un médicament tel que défini à l'une quelconque des revendications 4, 7, 9, 11, 13, 15, 17 ou 19, destinées à la prévention ou au traitement des manifestations liées au syndrome de sevrage spontané ou précipité des narcotiques ou mélanges de narcotiques.

**25)** Les compositions pharmaceutiques, renfermant à titre de principe actif au moins un médicament tel que défini à l'une quelconque des revendications 5, 8, 10, 12, 14, 16, 18, 20 ou 21, destinées à la prévention ou au traitement des manifestations liées au syndrome de sevrage spontané ou précipité des narcotiques ou mélanges de narcotiques.

**26)** Les compositions pharmaceutiques, renfermant à titre de principe actif le médicament tel que défini à la revendication 6, destinées à la prévention ou au traitement des manifestations liées au syndrome de sevrage spontané ou précipité des narcotiques ou mélanges de narcotiques.

Office européen des brevets

RAPPORT DE RECHERCHE EUROPEENNE

Numero de la demande
EP 95 40 0764

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,D | EP-A-0 057 115 (ROUSSEL-UCLAF)<br>* revendications * | 22-26 | A61K31/565<br>A61K31/575<br>A61K31/56 |
| A | BRAIN RES.,<br>vol. 598, no. 1-2, 1992<br>pages 343-8,<br>* page 347, colonne de gauche, ligne 6 - ligne 25 * | 1-21 | |
| A | BRAIN RES.,<br>vol. 269, no. 2, 1983<br>pages 297-302,<br>* page 297 * | 1-21 | |
| A | BRAIN RES.,<br>vol. 80, no. 1, 1974<br>pages 155-9,<br>* page 157, ligne 22 - ligne 24 * | 1-21 | |
| A | INT.J.IMMUNOPHARM.,<br>vol. 9, no. 4, 1987<br>pages 453-7,<br>* page 456, colonne de gauche, ligne 20 - colonne de droite, ligne 12 * | 1-21 | |

**DOMAINES TECHNIQUES RECHERCHES (Int.Cl.6)**

A61K

Le présent rapport a été établi pour toutes les revendications

| Lieu de la recherche | Date d'achèvement de la recherche | Examinateur |
|---|---|---|
| LA HAYE | 31 Juillet 1995 | Gerli, P |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)